# EUROPEAN PATENT APPLICATION

(11) **EP 1 405 908 A1**
(43) Date of publication of application: **07.04.2004**
(21) Application number: 02022194.1
(22) Date of filing: 04.10.2002
(51) Int. Cl.: C12N 15/09, C12N 15/11, C12N 15/85, C12N 15/90, C12N 5/10

(54) **Creation of high yield heterologous expression cell lines**

(71) Applicant: ProBioGen AG, 13086 Berlin (DE)
(72) Inventor: Sandig, Volker, c/o ProBioGen AG, 13086 Berlin (DE); Winkler, Karsten, c/o ProBioGen AG, 13086 Berlin (DE); Marx, Uwe, c/o ProBioGen AG, 13086 Berlin (DE); Wermelinger, Tobias, 8820 Wädenswil (CH)
(74) Representative: Helbing, Jörg, Dr. Dipl.-Chem.

(57) **Abstract**

The present invention relates to ubiquitous/universal processes for establishing cells capable of stable high yield expression of a recombinant gene, and for establishing stable universal precursor cells available for insertion of arbitrary target genes. The invention further relates to the cells obtainable by said processes.

## Description

### Technical Field

The present invention relates to ubiquitous/universal processes for establishing cells capable of stable high yield expression of a recombinant gene, and for establishing stable universal precursor cells available for insertion of arbitrary target genes. The invention further relates to the cells obtainable by said processes.

### Background of the Invention

Recombinant protein production is of central importance for different applications. Structural studies of proteins (rational drug design and drug optimisation are based thereon (Antivir. Chem. Chemother., 12 Suppl. 1, 43-49 (2001))), industrial applications of proteins (enzymes) and clinical use of recombinant proteins have increased the need for their efficient production. As of February 2000, according to a survey by the Pharmaceutical Research and Manufacturers of America, 122 biologics, including 20 monoclonal antibodies were either in phase III trials or awaiting FDA approval (K. Garber, 2001, Nature Biotech. 19, 184-185).
Depending on the application, native conformation and correct posttranslational modifications (such as glycosylation) of the recombinant protein are essential. Prokaryots such as the biotechnology "pet" organism *Escherichia coli (E. coli)* lack the ability to introduce posttranslational modification. Only eukaryotic cells possess the cell machinery necessary for co-translational and post-translational modifications as they are often required to produce functionally active proteins. Various eukaryotic systems for the production of a variety of heterologous proteins exist. Fungal expression systems, using e.g. derived from the genus *Saccharomyces, Candida, Pichia, Hansenula, Aspergillus* or *Kluyveromyces* are well established (Hollenberg and Gelissen (1997), Current Opinion in Biotechnology 8, 554-560). To circumvent the problem of plasmid instability sometimes encountered in fungi, sequences coding for heterologous proteins are ideally integrated into the fungal chromosome via homologous recombination.

Further problems encountered with fungal expression systems are overglycosylation of heterologous proteins and incorrect folding such as incorrect oligomerisation and insufficient ligand incorporation. Expression of heterologous proteins in insect cells - the DNA encoding the heterologous protein can also become integrated into the chromosome via recombination - gets around these problems. However, insect cells lack the ability to produce sialic acid and sialic glycans. Terminal sialic acid residues play divers biological roles in many glycoconjugates. Plants can also be used for the production of recombinant proteins. However, in these heterologous expression system difficulties in extraction and purification prove real bottlenecks.

Mammalian expression system, cultured cells as well as transgenic animals have none of these disadvantages. Recombinant proteins can be produced in cultured mammalian cells either transiently or constitutively (stably). For transient expression of recombinant a vector DNA encoding the recombinant protein is introduced into the cell and in general is not integrated into the cellular DNA. Expression titers of the recombinant protein are at the beginning high. However, since the vector DNA is generally not replicated, the vector DNA becomes diluted with each cell proliferation and hence the expression titer drops. Only rarely a vector DNA or part of the vector DNA illegitimedly recombines with the cellular genomic DNA and the gene encoding recombinant protein is stably integrated into the genome. If the gene encoding the recombinant protein is associated with a selection marker, cells carrying this cassette can be identified and isolated as stably transformed cells. Stable transformants have the advantage that the heterologous proteins are continuously produced. The expression titer is mainly determined by the strength of the promoter construct, the site of integration into the chromosome, the copy number and the type of recombinant protein in question. Many strong promoters are commercially available, however, their transcriptional activity varies depending on the cellular level of the relevant transcription factors and on the chromatin structure at the integration site. For example integration within the scaffod- or matrix attachment regions (S/MAR elements) of chromosomal DNA can augment the activity of promoters -and hence the expression of heterologous genes- and protect them from inactivation by the flanking chromatin. Therefore, it is highly desirable to chose a promoter highly active in a specific cell and to direct integration into an active part of the chromosome. Preferentially a single integration event is achieved, since heterologous genes at low copy number are in general expressed more stable than multicopy genes.

### Summary of the Invention

In view of the above, there is still a need for a method allowing the transformation/conversion of a cell line with an arbitrary gene coding for a product of interest to obtain a high yield recombinant protein producing cell, especially for a method without or only little cumbersome screening procedures. It was surprisingly found that high yield recombinant protein expressing cells are obtainable by first identifying a non-essential highly expressed cellular gene, in a transcriptional active part of the chromosome of a high yield expression starting cell; secondly directly replacing this cellular gene via homologous recombination with a first functional DNA sequence (e.g. by utilizing an appropriate targeting cassette) containing recombinase recognition sites (RRSs) for site-directed integration and optionally a "place-holder" gene comprising various functional sequences and selecting/isolating a stable clone of this precursor expression cell (functionalized cell); thirdly introducing the gene of interest (from here on called "target gene") coding for the target gene product (protein) by site-directed integration using a recombinase recognizing the RRSs incorporated with the first targeting cassette; and finally selecting/isolating a stable expression cell capable of producing large amounts of the recombinant protein.

It was moreover found that the above method is also applicable to mammalian hybridoma cells, e.g. human-mouse hetero-hybridoma cells such as H-CB-P1, which therewith allows the production of proteins having a mammalian/human glycosylation pattern.

Using the present invention it is possible to introduce stably any gene encoding a recombinant protein of interest into fungi, insect-, plant- and mammalian cells. Using the present invention the gene of interest encoding the recombinant protein will become integrated into the locus of a highly expressed cellular gene and preferably in close proximity to a highly active cellular promoter residing in an active part of the chromosome. Using the present invention precursor cell lines of various origin can be created carrying a place holder gene surrounded by RRSs. Using the present invention the place holder gene can be exchanged with the gene of interest, encoding the recombinant protein, by site-specific recombination at the RRSs catalyzed by a suitable recombinase, giving rise to the final high-yield expression cell.

More specifically, the present invention provides
(1) a process for preparing cells capable of stable high yield expression of a target gene product which method comprises
   (a) selecting a starting cell capable of stable high yield expression of a starting gene product being non-essential to the starting cell;
   (b) screening for the locus of the starting gene product within the genome of the starting cell;
   (c1) replacing the gene coding for the starting gene product with a first functional DNA sequence containing one or more recombinase recognition sites (RRS) to obtain a functionalized precursor cell; and
   (d) integrating a second functional DNA sequence containing a DNA sequence coding for the target gene product into the functionalized precursor cell obtained in step (c1) by use of a recombinase recognizing the RRSs incorporated with the first functional sequence, or
   (c2) directly replacing the gene coding for the starting gene product with a functional DNA sequence containing a DNA sequence coding for the target gene product;
(2) in a preferred embodiment of the method of (1) above the starting cell is an immortalized cell derived from B lymphocytes (preferably is a human-mouse hetero-hybridoma such as hetero hybridoma H-CB-P1 (DSM ACC 2104)) and integration of the functional DNA sequence(s) is effected at a Ig locus (preferably at a rearranged human Ig locus of said cell);
(3) a cell capable of high yield expression of a target gene,product obtainable by the method of (1) or (2) above;
(4) a method for preparing a functionalized cell comprising the steps (a) to (c1) as defined in (1) or (2) above;
(5) a precursor cell as defined in (4) above; and
(6) a method for high yield expression of a target gene product which comprises cultivating a cell as defined in (3) above.

### Description of the Figures

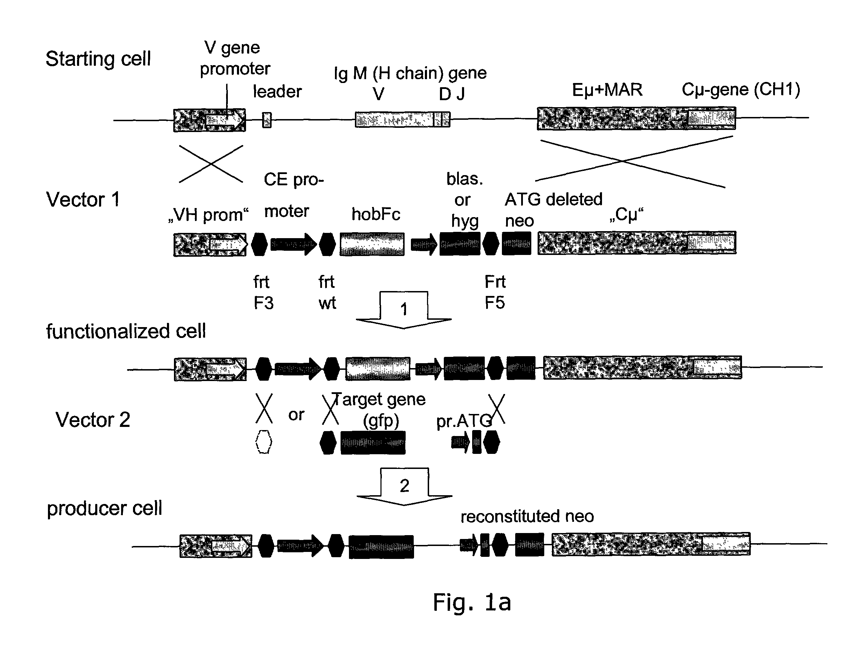

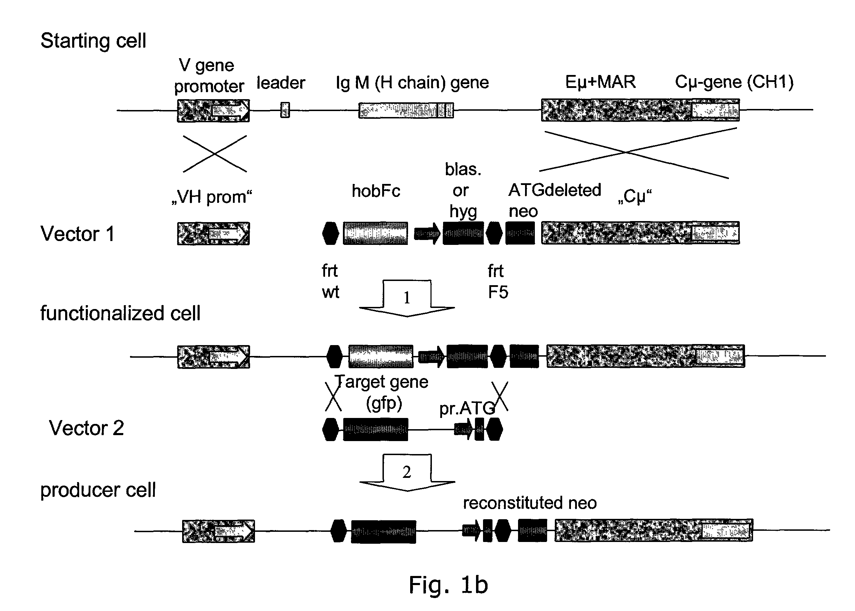

Fig. 1: Concept overview, multistep process to create high yield expression cell lines comprising site-specific integration of genes into an IgH locus at frt sites.
The IgH locus of H-CB-P1 is represented in the upper graph of figures 1a and 1b. It contains the variable gene promoter followed by a protein leader sequence and specific V, D, and J genes rearranged and positioned next to the enhancer Eµ, the MAR and the Cµ coding sequences. Target sequences for homologous recombination are shown marmorate. Via homologous recombination between the flanking sequence elements "Vhprom" and "Cµ" of vector 1 (targeting vector) and the genomic DNA, first functionalized sequences located between the flanking sequences are introduced into the genomic DNA and a recombinant PBG03 genome is the result. The first functionalized sequence contains frt sites (frtF5 frtF3 and frt wt), an artifical strong promoter CES (Fig. 1a) or no additional promoter (Fig. 1b) upstream of the first expressed gene(hobFc). In addition a blasticidine or hygromycin resistance gene and an ATG deleted neomycin gene are part of the first functionalized sequence. The recombinant genome carries the first functional sequences integrated in the chromosomal DNA.

FLP recombinase catalyses recombination at frtF5 and frtF3 or at frt wt and frtF3 sites of the recombinant H-CB-P1 genome and vector 2, the actual gene of interest is introduced and expressed from the artificial or the endogenous VH promoter (Fig. 1a and 1b, respectively). Parts of the first functionalized sequence located between frtwt and frt F3 sites are replaced by a weak promoter followed by an ATG which after recombination is positioned in the same open reading frame as the ATG-deleted neomycin gene. The resulting genome has lost the hobFc gene and the blasticidin or hygromycin resistence genes and instead has the gene of interest (target gene) and a functional neo gene.

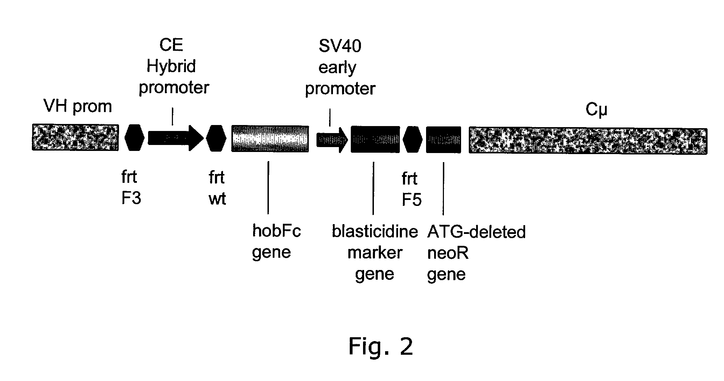

Figure 2: The endogenous cassette (CEShobFcblas) of the targeting vector.
The detailed structure of the endogenous cassette containing an CES promoter, a hobFC fusion gene, a blasticidin resistance gene and a start codon (ATG) deficient neomycin gene is shown. In more detail, the endogenous sequence contains a modified frt site (frtF5) followed by a hybrid promoter structure comprising the early CMV promoter/enhancer elements as well as the first intron of the elongation factor alpha gene. The next element is a frt wildtype site followed by the hobFC fusion gene (hobFC), and the SV40 polyadenylation signal (SV40PA). A weak SV40 promoter controlling the expression of the blasticidin resistance gene follows. The last elements are a modified frt site (frtF3) and next to it the ATG deficient neo gene. Modified frt sites F3 and F5 allow recombination with identical sites but not with wildtype frt sites and F5 or F3 sites respectively. The frt F3 site is positioned to upstream of the neo gene to form a contiguous open reading frame lacking the ATG.

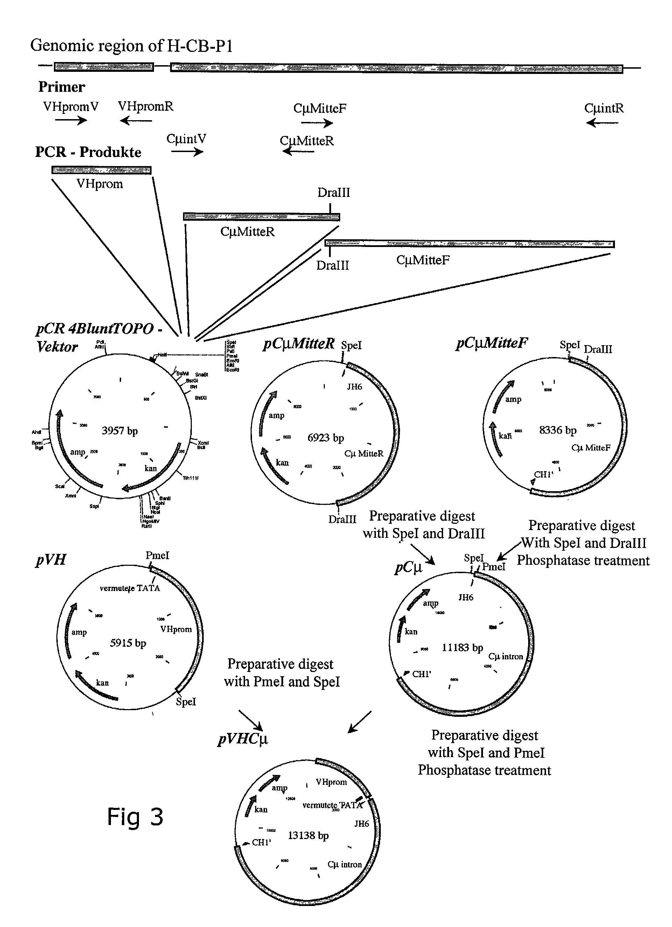

Fig. 3: Cloning strategy for the intermediate vector pVCµ containing the flanking regions Vhprom and Cµ.
The 2 kb VH promoter sequence was amplified from PBG03 cell genomic DNA using forward primer VHpromF (SEQ ID NO:1) and reverse primer VHpromR (SEQ ID NO:2). The PCR product VHprom was cloned into the pCR 4BluntTOPO vector (Invitrogen) and the resulting vector named pVH. The 7.4 kb Cµ region was amplified from genomic H-CB-P1 DNA as two overlapping fragments, namely CµMitteR and CµMitteF. The primers CµintV (SEQ ID NO:3) and CµMitteR (SEQ ID NO:5) give rise to the product CµMitteR and the primers CµMitteF (SEQ ID NO:6) and the reverse primer CµintR (SEQ ID NO:4) produce the fragment CµMitteF. Both fragments were cloned into a pCR 4BluntTOPO vector (Invitrogen) and the resulting vectors called pCµMitteR and pCµMitteF. The full length Cµ sequence was re-established by opening both vectors with the restriction enzyme SpeI and DraIII, and ligating the SpeI-DraIII fragment from pCµMitteR into the opend pCµMitteF. The resulting vector pCµ carries the full-length Cµ region (Cµ intron). The VH promoter sequence and the Cµ sequence were combined in one vector pVHCµ, by digesting both pVH and pCµ with the restriction SpeI and PmeI, and inserting the isolated Vhprom PmeI-SpeI fragment into the phosphatase treated opend vector pCµ.

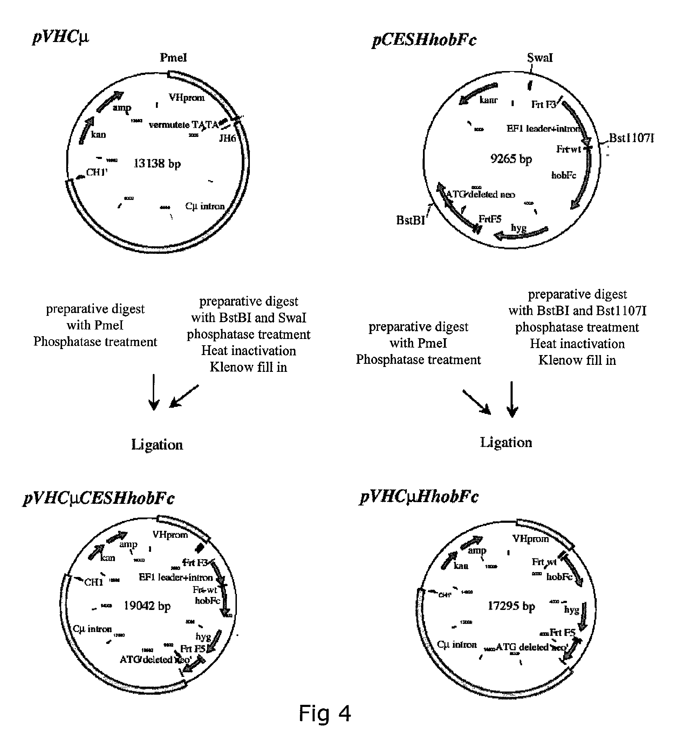

Fig. 4: Cloning strategy for the targeting vectors pCESHhobFc and pVHCµHhobFc.
The targeting vector pVHCµCESHhobFc containging the highly active promoter CES and the hobFC fusion gene was created by ligating an end-filled SwaI-BstBI fragment isolated from pCESHhobFc into the pVHCµ vector that had been digested with PmeI and dephosphorylated. The targeting vector pVHCµHhobFc that has the hobFC fusion gene but no CES promoter, was prepared by ligating an endfilled Bst1107-BstBI fragment isolated from pCESHhobFc into an PmeI digested and dephosphorylated vector pVHCµ.

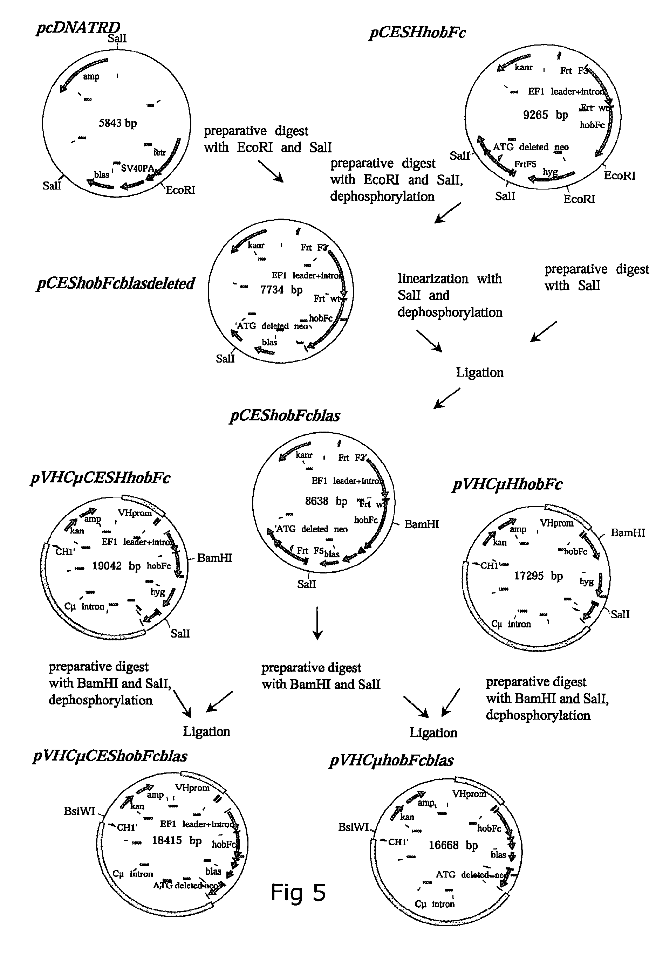

Fig. 5: Cloning strategy for targeting vectors carrying a blasticidin resistant gene, pVHCµCEShobFcblas and pVHCµhobFcblas.
The plasmid pcDNATRD was used as donor for the blasticidin gene. To delete the hygromicin gene sequences as well as the FRT5 and neomycin sequences from the vector pCESHhobFc, the vector was digested with EcoRI and SalI and dephosphorylated. An EcoI-SalI fragment from pCDNATRD containing the blasticidin resistant gene sequence was ligated into the previously opened pCESHhobFc vector and the resulting plasmid named pCEShobFcblasdeleted. The Frt F5 sequence and the ATG-deleted neomycin sequence were isolated from pCESHhobFc as a SalI SalI fragment and re-inserted into the SalI site of pCEShobFcblasdeleted. The resulting plasmid pCEShobFcblas was used together with pVHCµCESHhobFc to create pVHCµCEShobFcblas. The BamHI-SalI fragment comprising the hobFc sequence and the blasticidin gene was isolated from pCEShobFcblas and inserted into vector pVHCµCESHhobFc opened with BamHI and SalI, giving rise to pVHCµCEShobFcblas. The vector pVHCµhobFcblas was created by ligating a BamHI-SalI fragment containing the hobFc gene and the blasticidin gene into vector pVHCµHhobFc digested with BamHI and SalI.

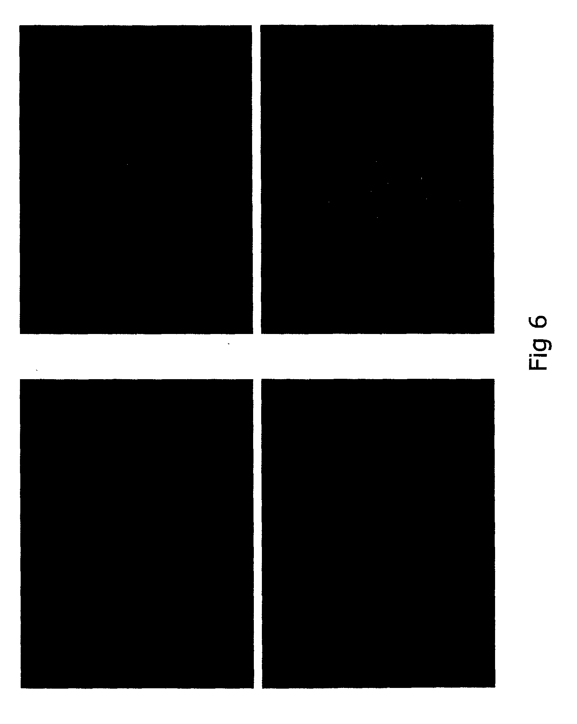

Fig. 6: Immunostaining of H-CB-P1 clones
H-CB-P1 clones obtained through transfection of H-CB-P1 cells with pVHCµ CESHhobFcblas were immunostained with a Texas Red conjugated anti-human IgG, Fy fragments specific antibody isolated from goats or an AMCA conjugated anti-human IgM, Fc_{5µ} specific antibody isolated from goats. The left column shows two H-CB-P1 clones stained with the Texas Red conjugated antibody and visualized with an UV WG filter. In the right column the same clones are shown after staining with the AMCA conjugated anti-IgM antibody and visualized under UV filter WU. Whereas for the clone in the upper panel only IgG staining is evident, staining with both antibodies is present for the clone in the lower panel. The first clone may result from homologous recombination whereas the other clone contains an illegitimate insertion of the functional sequences.

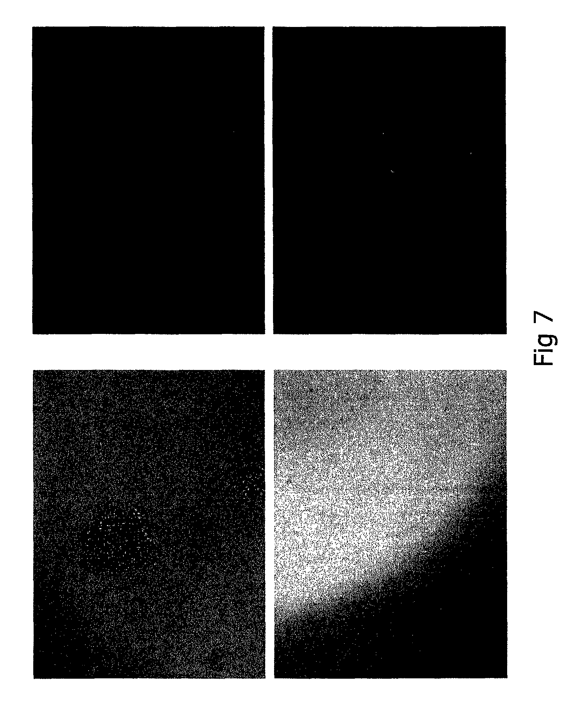

Fig. 7: Direct immunostaining of H-CB-P1 clones and further expansion of clones.
It is demonstrated that H-CB-P1 clones could be immunostained without jeopardizing the cells viability and that subsequent expansion of the stained clone was possible. A H-CB-P1 clone cultured for ten days in a 96 well plate was immunostained with an Texas Red conjugated anti-IgG antibody. A picture of the clone prior to immunostaining visualized with normal light microscopy, is shown in the top left panel. The same clone immunostained with the Texas Red conjugated anti-IgG antibody is shown in the top right hand panel. The bottom panels show pictures of the well post trypsinisation. No cells can be seen when the picture was taken under a normal light microscope, as shown in the bottom left hand panel. The right hand panel shows the same well examined under UV filter WU. The cells are completely removed from the well and the fluorescent antibody precipitate remained in the well and is not attached to the cell surface.

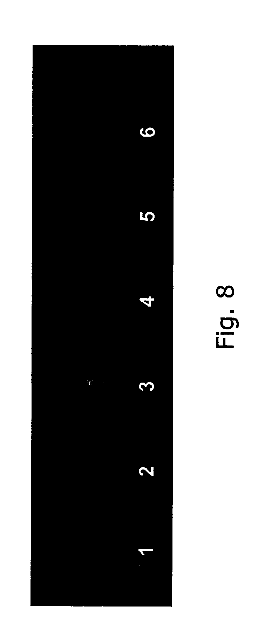

Fig. 8: Ant-IgM dot blot of cell culture supernatants from induvidual clones
Supernatant of the following clones
1: pVHCµhobFcblas-D6; 2: pVHCµhobFcblas-G8;
3: pCEShobFcblas-A3; 4: pVHCµCEShobFcblas-B4;
5: pVHCµCEShobFcblas-D3; 6: pVHCµCEShobFcblas-G8 were spotted onto a membrane and subjected to the ECR staining method. Since the starting cell population homogenously produces IgM, clones with no detectable IgM expression result from inactivation of the IgM H gene mediated by the targeting vector. The clone A3 generated by transfection of pCESHhobFcblas which lacks homologous flanking sequences was unable to target the IgM locus and expresses IgM.

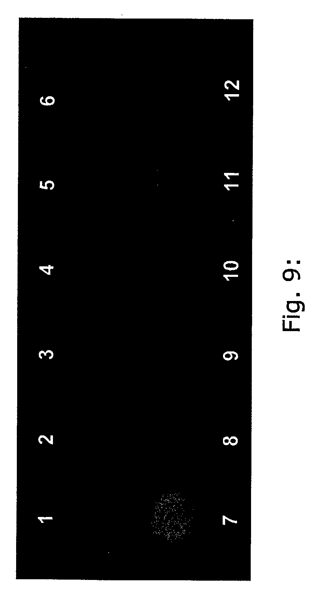

Fig. 9: Anti-IgG dot blot of cell culture supernatants from individual clones.
Supernatant from the following clones
1: pVHCµhobFcblas D6; 2: pVHCµhobFcblas D6 (1:2 diluted); 3: pVHCµhobFcblas-G8; 4: pVHCµhobFcblas G8 (1:2 diluted); 5: pCEShobFcblas A3; 6: pVHCµCEShobFcblas B4; 7: pVHCµCEShobFcblas D3; 8: pVHCµCEShobFcblas D3 (1:2 diluted);
9: pVHCµCEShobFcblas D3 (1:10 diluted); 10: pVHCµCEShobFcblas G8;11:hobFc standard 500 ng/ml; 12: hobFc standard 50 ng/ml IgG
were spotted onto a membrane and subjected to the ECR staining procedure using an anti-IgG antibody.

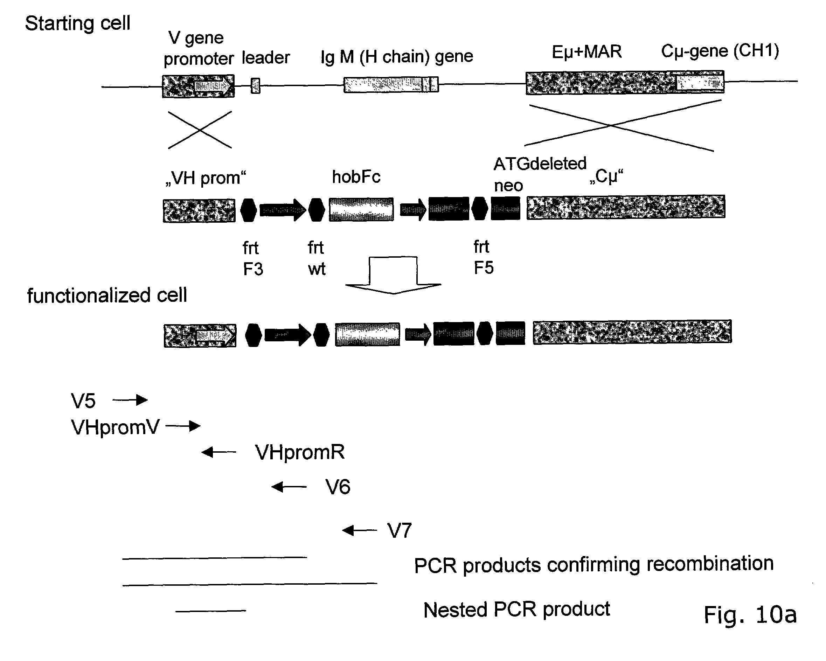

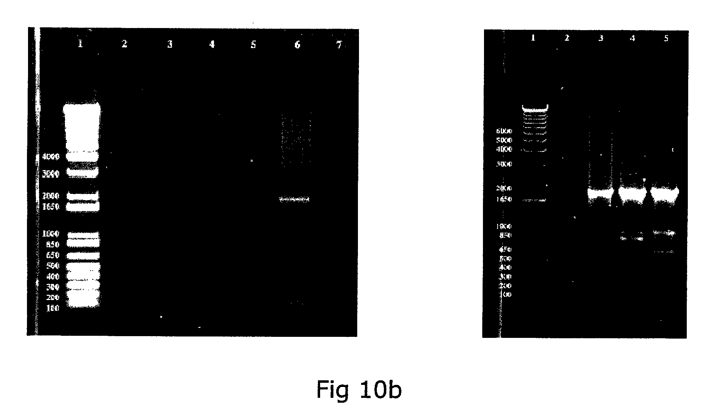

Fig. 10: Detection of homologue recombination via PCR
To test whether a homologue recombination event had occurred between the (targeting) vector and the Ig locus of H-CB-P1 cells, a PCR strategy was applied. Upon recombination the endogenous cassette of the vector containing the CES promoter, the hobFc gene and a resistance gene (hygromycin or blasticidin) followed by the ATG-deleted neomycin gene becomes integrated between the genomic V gene promoter sequences and the enhancer Eµ. The forward primer V5 (SEQ ID NO:7) which binds to the genomic V gene promoter sequences outside of the fragment Vhprom was combined with primers V6 or V7 (SEQ ID NOs:8 and 9, respectively), which bind specifically within the first functional sequence. The occurrence of PCR products is strictly dependent on co-localisation of both primer binding sequences and hence homologous recombination. To confirm any positives the putative positiv PCR product was used in a nested PCR reaction with the primers VHpromF and VHpromR (SEQ ID NOs:1 and 2, respectively).
a: primer position, b: electrophoretic analysis of PCR products
left: first PCR V5, V7
lane 1: 1kb ladder (Invitrogen); lane 2: clone pVHCµhobFcblas H6;
lane 3: clone pVHCµCEShobFcblas B10; lane 4: clone pVHCµhobFcblas D4;
lane 5: clone pVHCµhobFcblas D8; lane 6: clone pVHCµhobFcblas E11;
lane 7: neg control H-CB-P1
right: nested PCR
lane 1: 1kb ladder (Invitrogen); lane 2: neg control H-CB-P1;
lane 3: clone pVHCµCEShobFcblas H6; lane 4: clone pVHCµhobFcblas D4;
lane 5 clone pVHCµhobFcblas E11

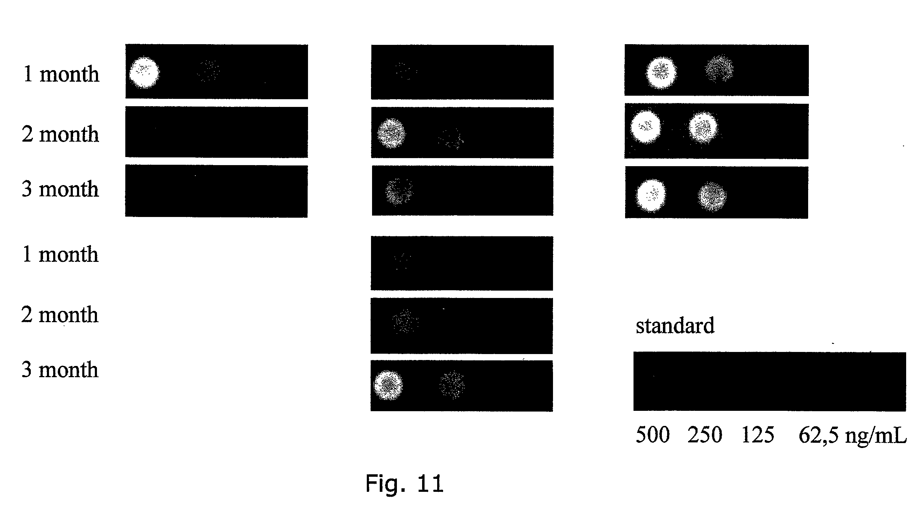

Fig. 11: hobFc expression in the absence of selction pressure.
Cells were cultivated for 3 month in the absence of selection pressure. To determine expression cells were seeded at a density of 10⁵ cells/ml. After 24h cell culture supernatants were harvested and subjected to a westen blot (dot blot) using an anti human Fc antibody. Supernatants were applied to the filter from left to right: undiluted, 1:2 dilution and 1:10 dilution.
Left: clone pCEShobFcblas A3 (random insertion),
Center top pVHCµhobFcblas G8; Center bottom pVHCµhobFcblas G8;
Right pVHCµCEShobFcblas D3.
Expression is stable in clones resulting from homologous insertion of the functionalised sequences including the hobFc gene.

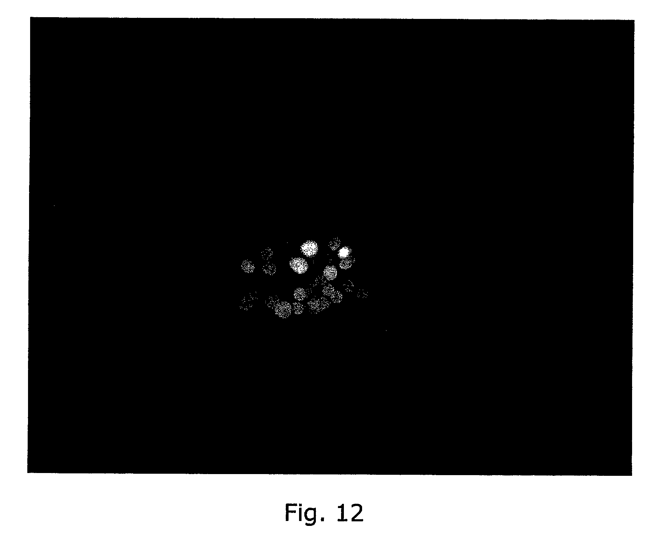

Fig 12: Generation of a target cell clone using GFP as a model target gene.
Clone pVHCµCEShobFcblas D3 (renamed PBG04) was transfected with vector 2 comprising a second fuctional sequence ( frtwt, GFP open reading frame and polyadenylation signal, minimal promoter followed by ATG and frtF5) and plasmid pflp comprising a functional expression unit for the flp recombinase (Note: the vector is unable to express GFP in a naive cell because it lacks a functional promoter driving expression). After two weeks of selection with G418 individual stable clones strongly expressing GFP are detectable. GFP expression as well as G418 resistance depends on the homologous recombination event.

### Detailed Description of the Invention

The present invention provides a method to transform any starting cell, in particular any starting eukaryotic cell into a stable high yield expressing cell. To achieve continuous recombinant expression, the gene encoding the recombinant product becomes integrated into the genomic cellular DNA. Expression levels are highly determined by the site of integration of the recombinant gene into the cellular DNA. Therefore, the here presented method comprises the integration of a recombinant gene into a transcriptionally highly active part of the genome of a cell. The gene of interest coding for the recombinant protein can either be under the control of very strong recombinant promoter, or be placed under the control of a highly active cellular promoter by integrating it downstream of the highly active cellular promoter.

In a preferred embodiment of the method (1) of the invention the starting cell secretes the starting gene product, preferably in an amount of at least 0.3 fmol/cell/d of a polypeptide chain (which equals 30pg/cell /day for a protein of approximately 90kd) and more preferably in an amount of more than 1fmol/cell/d (which equals 100pg/cell /day for a protein of approximately 90kd). Alternatively, in case the starting cell does not secrete the starting gene product a gene coding for a highly expressed preferably nonessential intracellular or membrane protein or a highly expressed noncoding RNA is selected.

In another preferred embodiment, the starting cell is a eukaryotic cell, preferably a mammalian cell, most preferably a primary, immortalized or fusionated cell or a genetic modification thereof. Thus, the starting cell may be selected from primary cells, immortalized cells or tumor cells or genetic modifications thereof, cell hybrids, cell lines used generally in protein manufacturing such as NS0, CHO, BHK, HEK293, preferably it is a mammalian hybridoma or hetero-hybridoma cell (e.g. human-mouse, human-rat or the like) and most preferably is human-mouse hetero-hybridoma H-CB-P1 (DSM ACC 2104; previously referred to as ZIM517).

The starting gene product is preferably selected from secreted proteins such as antibodies, cytokines, hormones, enzymes, transport proteins storage proteins, structural proteins, etc. The starting gene product is either known a main product of the chosen starting cell. So stable expression of IgM has been observed for H-CB-P1 or selected in a screening procedure. Screening may be based on individual or combined methods comprising microarray expression analysis, 2D protein gel electrophoresis, quantitative PCR, RNAse protection, northern blot, ELISA and western blot. The power and sensitivity of these individual methods is known to those skilled in the art.

The method of the invention allows the production of any recombinant protein. Preferred target gene products include, but are not limited to, enzymes, in particular proteases, protease inhibitors, hormones, cytokines, receptors or soluble forms thereof (e.g. receptors lacking transmembrane or intracellular domains), full-length antibodies or antibody domains and fusion proteins combining domains of these protein classes.

In a first option of embodiment (1) comprising steps (a), (b), (c1) and (d), the replacement of the starting gene is effected by an one step replacement strategy, wherein the starting cell is contacted with a vector construct containing the first functional sequence, said first functional sequence inactivating and partially or completely replacing the gene coding for the starting gene product. Alternatively, the replacement is effected in a two or multistep strategy, wherein the gene coding for the starting gene product is deleted or inactivated and subsequently contacted with a vector containing the first functional sequence, said first functional sequence being incorporated at the site of the deleted/inactivated starting gene product.
Specific incorporation of the first functional sequence at the location of the starting genes is facilitated by sequences flanking the first functional sequence in the vector which are homologous to the target gene or adjacent sequences. These flanking sequences are obtained either from lambda, cosmid, pac or bac libraries of the starting cell or generated by PCR using starting cell DNA as a template.
The percentage of cell clones resulting from specific incorporation of the first functional sequence at the location of the target gene may be further increased by employing a dual selection strategy, where a positive selection marker is contained as part of the first functional sequence and a negative selection marker separated from the first functional sequence by a homologous flank. Homologous exchange allows incorporation of the positive selection marker in the absence of the negative selection marker. Examples for positive selection markers are the hygromycin, blasticidin, neomycin, or glutamin synthetase genes and the HSV tk or the Cytosine desaminase gene are negative selection markers. Markers and methods for their application are known to those skilled in the art.
Cell clones resulting from homologous exchange are identified by the presence of elements of or gene products expressed from the first functional sequence and the inactivation of at least one allele of the starting gene. These cell clones represent the functionalised precursor cell.

The first functional sequence comprises one or more RRS(s) selected from loxP, frt, att L and attR sites of lambdoid phages, recognition sites for resolvases or phage C31 integrase. The first functional sequence may further comprise sequences selected from marker sequences, secreted protein genes, promoters, enhancers, splice signals, polyadenylation signals, IRES elements, etc.

To create a producer cell for the target gene product, the functionalized precursor cell (if not already a producer as obtained in step (c2) of second option of embodiment (1), see below), e.g.the PBG03 clone D3 (DSM ACC2577), is subsequently contacted with a second vector containing the second functional sequence. The second functional sequence comprises the target gene and RRS(s) for said recombinases present in the first functional sequence. The second functional sequence further comprises functional sequences selected from promoter sequences, marker sequences, splice donor and acceptor sequences, recombinase recognition sequences differing from RRS of the first functional sequence, etc.

The integration of the second functional DNA sequence is effected by recombinases recognising the RRS with or without accessory proteins (e.g., Cre, Flp, φC31 integrase, resolvase and the like). These recombinase and accessory proteins, mRNA coding for these proteins or viral or nonviral vectors allowing there transient expression are delivered together with, shortly before or after delivery of the second functional sequence.

A pure population of clones containing the second functional sequence at the location of the starting gene is achieved by selection using a reconstituted functional selection marker gene. As an example an inactive ATG deleted selection marker gene introduced with the first functional sequence may be reconstituted by delivery of an active promoter and an in frame-ATG codon with the second functional sequence.

In a second option of embodiment (1) of the invention (comprising steps (a), (b) and (c2)) the gene coding for the starting gene product may directly (i.e. without the provision of the precursor cell) be replaced with a functional DNA sequence containing a DNA sequence coding for the target gene product (hereinafter shortly referred as "third DNA sequence"). Said third DNA sequence may be incorporated by a one- or multi-step strategy as described herein before. The third DNA sequence may further contain functional sequences (such as promoters, markers etc.) as the first and second DNA sequence described herein before. This second option of embodiment (1) of the invention is particularly preferred, if only one target gene product is to be produced so that the generation of the precursor cell is not necessary.

In preferred embodiment (2) of the invention, the starting cell preferably is a human-mouse hetero-hybridoma cell, preferably is hetero-hybridoma cell H-CB-P1 (DSM ACC2104). The integration of the functional DNA sequence is effected at a Ig locus, preferably at one of the human rearranged Ig loci (e.g. light chain or heavy chain (λ or κ)) of the hybridoma cell. The rearranged immunoglobin locus is the genomic sequence surrounding the functional Ig gene (heavy chain λ or κ ) modified from the germ line chromosomal configuration during maturation of the B-lymphocyte which gave rise to the hybridoma. The IgH locus is located at chromosome 14q32.33. In H-CB-P1 this locus is formed by the rearranged and affinity matured VH1-2 gene linked via a D-gene to the J_{H}6-gene linked via the µ-intron to the Cµ sequences (DD 296 102 B3). The sequence of the rearranged VDJ region of the H-CB-P1-IgH locus is provided in SEQ ID NO:12

The present invention is further explained by the following examples which are, however, not to be construed to limit the invention.

The cell line HCB-P1 was deposited at the "Zentralinstitut für Molekularbiologie, Akademie der Wissenschaften der DDR", Robert-Rossle-Str. 10, Berlin Buch, DDR-1115 as ZIM-0517 on March 16, 1990 and was transferred to the DSMZ, Deutsche Sammlung von Mikroorganismen und Zellkulturen GmbH, Maschroder Weg 13, 38124 Braunschweig, Germany, on December 12, 2000 and here given the depositary number DSM ACC 2104. The PBG03 clone D3 (pVHCµCES hobFcblas) was renamed PBG04 and was deposited at the DMSZ as DSM ACC2577 on September 18, 2002.

### Examples

### Materials and Methods

### Materials:

### DNA Cloning Techniques

### Isolation of Genomic DNA:

Cells from a T25 cm² flask were trypsinized (see chapter "Trypsinisation" below), the resuspended cell pellet transferred into a 1.5 ml Eppendorf tube and 200 µl PBS added. The tube was centrifuged for 5 min at 13,200 rpm, the supernatant discarded and the pellet re-suspended in 2 ml of solution A. After transfer of the suspension into a Falcon tube (15 ml), 133 µl 10% SDS and 333 µl protease K were added. Either a 3 h incubation at 55°C, or an over night incubation at room temperature followed. The suspension was mixed with 607 µl 6 M NaCl and vortexed for 15 s before centrifuging it (4300 rpm, 4°C, 20 min). The supernatant was transferred into a Falcon tube (15 ml) and mixed with 2.5 ml 100% Ethanol. A threadlike DNA precipitate formed at the interface and was removed with a pipette tip and re-suspended in ½ TE buffer. The DNA was allowed to completely dissolve at 56°C before it was stored at 4°C.

### PCR:

The PCR method was used to isolate genomic DNA sequences (preparative PCR) or to detect certain DNA sequences (analytical PCR).

### Preparative PCR:

Preparative PCR reactions (50 µl) were set up with the Expand High Fidelity PCR kit (Roche) according to the manufacturer' s instruction (20-30 ng of template, 5 µl of 15 mM Mg Cl₂ buffer (10x), 5 µl dNTP mix, 0.5 µl of each primer (30 nM), 0.5 µl polymerase and filled to 50 µl with water). The PCR products were purified using a QIAquick PCR purification kit (QIAGEN).

### Analytical PCR:

Analytical PCR reactions (10 µl) were prepared with Taq polymerase kit (QIAGEN) following the manufacturer's instruction (10 ng of template, 1 µl 10x buffer, 0.5 µl dNTP mix, 0.1 µl of each primer (30 pM), 0.1 µl Taq polymerase and filled to 10 µl with water).

The PCR cycling program varied for each product according to the annealing temperature of the primer (see Table 2) and the length of the expected PCR products (determined elongation time and temperatur; see Table 1).

**Table 1:**

| Length of the amplified fragment determines elongation time | | | | | |
|---|---|---|---|---|---|
| parameter | length, time temperature | length, time temperature | length, time temperature | length, time temperature | length, time temperature |
| length of fragment | < 750 bp | 1.5 kb | <3 kb | > 3 kb, | 6 kb |
| elongation time | 45 s | 1 min | 2 min | 3 min + 15 s after each step | 4 min + 15 s after each step |
| temperature | 72°C | 72°C | 72°C | 68°C | 68°C |

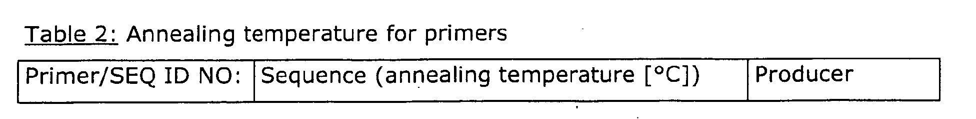

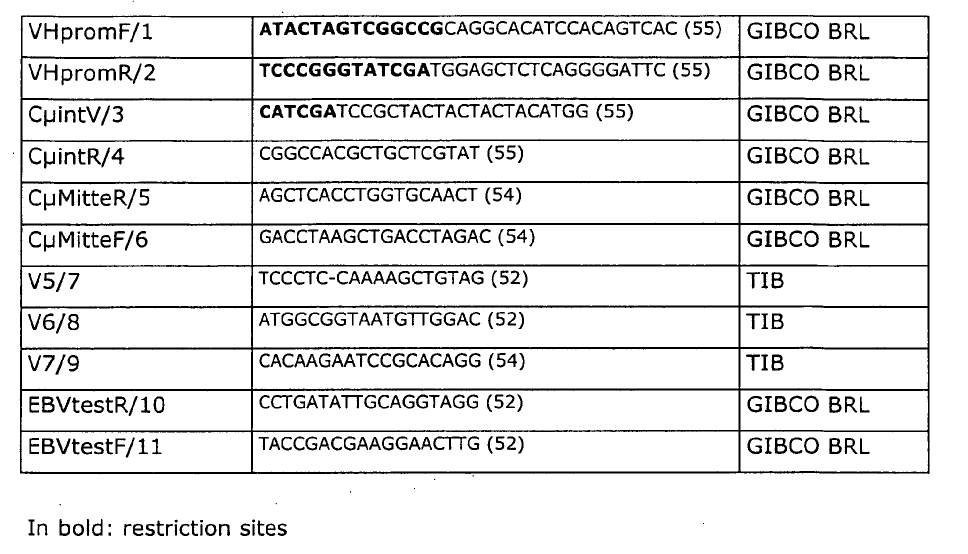

### Amplification, isolation and quantification of plasmid DNA:

*E. coli* transformants were grown in a 1 ml, 30 ml or 100 ml culture and plasmid DNA isolated using Mini-, Midi- or Maxi- plasmid purification kits (QIAGEN), respectively. The instruction of the manufacturer were followed. The DNA concentration was determined via spectroscopy, measuring the absorbance at 260 and 280 nm.

### Restriction Enzyme Digestion:

Plasmid DNA was digested with 1 unit of the appropriate restriction enzyme for 1 µg of DNA, using the buffer and temperature recommended by the supplier (see Table 3). If the analysis required the use of two or more restriction enzymes, the reactions were carried out simultaneous digestion if possible. Otherwise, sequential single digestions were performed with an intervening column purification step (QIAGEN) of the reaction mix.

**Table 3:**

| Used Restriction Enzymes | | | | | | |
|---|---|---|---|---|---|---|
| Enzyme | Conc. | Temp. | Buffer | Inact. | Producer | Art. No. |
| BamHI | 20 U/µl | 37°C | 2^{a} | 65°C | BioLabs | R0136L |
| BgIII | 40 U/µl | 37°C | M | 65°C | Boehringer | 1175068 |
| BsaBI | 10 //µl | 60°C | 2 | 80°C | BioLabs | R0556S |
| BsiWI | 10 U/µl | 55°C | 3 | 80°C | BioLabs | R0136L |
| Bst11071 | 5 U/pl | 37°C | 3 + BSA | 80°C | BioLabs | R0553S |
| BstBI | 20 U/µl | 65°C | 4 + BSA | 80°C | BioLabs | R0519S |
| ClaI | 10 U/µl | 37°C | H | 65°C | Roche | 404217 |
| DraIII | 20 U/µl | 37°C | 3 + BSA | 65°C | BioLabs | R0510L |
| EagI | 10 U/µl | 37°C | 3 + BSA | 65°C | BioLabs | R0505S |
| EcoRI | 40 U/µl | 37 °C | H | 65°C | Roche | 200310 |
| HincII | 10 U/µl | 37°C | 3 + BSA | 65°C | BioLabs | R0103S |
| HindIII | 40 U/µl | 37°C | B | 5°C | Roche | 798983 |
| KpnI | 10 U/µl | 37°C | L | 65°C | Roche | 899186 |
| Mfel | 10 U/µl | 37°C | 4 | 65°C | BioLabs | R0589S |
| NotI | 10 U/µl | 37°C | 3 + BSA | 65°C | BioLabs | R0189L |
| PmeI | 10 U/µl | 37°C | 4 + BSA | 65°C | BioLabs | R0560L |
| PstI | 10 U/µl | 37°C | H | 80°C | Roche | 621633 |
| PvuII | 5 U/µl | 37°C | 3 + BSA | 65°C | BioLabs | R0150S |
| SacII | 20 U/µl | 37°C | 4 + BSA | 65°C | BioLabs | R0157S |
| SalI | 10 U/µl | 37°C | H | 65°C | Boehringer | 567663 |
| ScaI | 10 U/µl | 37°C | H | 80°C | Roche | 775266 |
| SmaI | 20 U/µl | 37°C | 4 + BSA | 65°C | BioLabs | R0141S |
| SpeI | 10 U/µl | 37°C | 2 + BSA | 65°C | BioLabs | R0133L |
| SspI | 5 U/µl | 37°C | 2 + BSA | 65 | BioLabs | R0182L |
| StyI | 10 U/µl | 37°C | H | 65°C | Roche | 85111023 |
| SwaI | 10 U/pl | 25°C | 3 + BSA | 65°C | BioLabs | R0604L |
| XbaI | 10 U/µl | 37°C | H | 65°C | Boehringer | 674265 |

| | | | | | | |
|---|---|---|---|---|---|---|
| a. specific buffer | | | | | | |

### End repair of DNA with 5' protruding termini:

To "blunt" 5' overhangs such as those produced by EcoRI, the digested DNA was treated with the Klenow fragment (Roche) according to the manufacturer's instruction. The endfilling reaction was stopped by a heat inactivation step (65°C, 20 min) and the DNA ethanol precipitated or directly subjected to gel purification.

### Dephosphorylation of vector DNA:

To prevent self-ligation of linearised vector DNA (see passage "Ligation" below) with compatible ends, DNA was dephosphorylated using alkaline phosphatase (AP) (Roche) according to the manufacturer's instruction. The AP was heat inactivated (65°C, 15 min) and the DNA gel purified (for electrophoresis see passage "Agarose Gel Electrophoresis" below) prior to use in a ligation reaction.

### TOPO Cloning:

PCR amplification products were cloned into TOPO vectors from Invitrogen. According to the instruction of the TOPO cloning kit, the purified PCR product (0.25-2 µl ) was mixed with the salt solution (0.5 µl), water added to reach a volume of 2.5 µl and then the TOPO vector (0.5 µl) added. Following a 30 min incubation at room temperature, the reaction tube was transferred onto ice, 2 µl of the reaction added to "one shot chemically competent E. coli" and the cells incubated for 30 min on ice. The cells were heat-shocked (42°C, 30 s), immediately transferred back onto ice and 250 µl of room temperature SOC medium added. The transformation reaction was incubated for 1 h at 37°C with shaking (300 rpm) before the mixture was plated on LB plates containing either Kanamycin or ampicillin. The plates incubated of overnight at 37°C.

### Ligation:

All ligation reactions were carried out in 10 µl volumes with 0.1-1 µg of dephosphorylated vector and an excess of insert. The reaction contained 2 µl T4 ligation buffer (Gibco BRL) and 1 µl T4 ligase (Roche), and were incubated for two hours at 16°C or overnight at 4°C. The ligation reaction was transformed into bacteria.
To reduce the level of unwanted non-recombinants, ligations could be post-digested with a suitable restriction enzyme if there was a unique restriction site in the self-ligated vector. Following the digestion, the ligation reaction was ethanol precipitated in the presence of acrylamide (centrifugation at 4°C, 14,000 rpm, 15 min) before the re-dissolved DNA was used again in a transformation reaction

### Transformation of competent bacteria:

Competent *E. coli* XL2 (stored at -70°C) were thawed on ice, mixed with either the ligation reaction (also kept on ice, see passage "Ligation" above) or with 1-100 ng plasmid DNA (re-transformation) and incubated on ice for 20 min. Subsequently the transformation reaction was heat-shocked (30-60 s, 42°C), the tube returned onto ice and 205 µl SOC medium (free of any antibiotic) added and the reaction incubated shaking (300 rpm) at 37°C for 45 min. The transformation reaction was plated on LB plates containing an antibiotic (either kanamycin (40-60 µg/ml) or ampicillin (50-100 µg/ml)) and incubated overnight at 37°C. The bacterial colonies were counted and the efficiency of the transformation reaction calculated.

### Agarose Gel Electrophoresis:

DNA fragments were separated according to their length on 0.7-1.5% agarose gels. The agarose was dissolved in 1xTAE buffer and 2 µl ethidiumbromide/100 ml agarose added. When the agarose had dissolved, it was poured into a tray and allowed to set. The DNA sample was mixed with the loading buffer Orange G, loaded onto a horizontal gel and run at 40-90 V with 1xTAE as running buffer. The DNA/ethidium bromide complexes were visualized under UV light.

### Gel Purification of DNA Fragments:

The DNA was separated on an agarose gel (40-80 V) and DNA bands (visualised under UV light) of interest excised with a scalpel. Using a QIAquick gel extraction kit (QIAGEN), the DNA was extracted from the agarose block according to the manufacturer's instructions.

### Cell culture

### Trypsinisation:

Adhesive cells were harvested using trypsin. First the culture medium was removed and the cell monolayer washed with citrate buffer (pre-warmed to 37°C). A small amount of trypsin was added directly to the cell monolayer and incubated for 3-5 min at 37°C. Trypsinisation was stopped by addition of PBG 1.0 medium supplemented with 5% FCS (see Table 4). The cell suspension was transferred into a Falcon tube (50 ml) and centrifuged for 10 min (800 rpm, 30°C). The cell pellet was re-suspended in fresh medium and the cells used for electroporation or for further passaging.

**Table 4:**

| Volumes of citrate, trypsin and PBG 1.0 supplemented with 5% FCS used for the trypsinisation of the cells growing in different flasks | | | |
|---|---|---|---|
| Tissue culture flask | Citrate buffer (ml) | Trypsin (ml) | PBG 1.0/5%FCS (ml) |
| T25 | 1.0 | 0.5 | 4.5 |
| T85 | 2.0 | 1.0 | 9.0 |
| T180 | 5.0 | 2.0 | 8.0 |

### Counting of Cells

After the cells had been trypsinized they were counted in a Neubauer chamber (haematocytometer). A small volume of the cell suspension was introduced into the chamber and the chamber placed under a microscope. Only cells within one of the four squares of the chamber were counted, the cell number multiplied with the factor 10⁴ to obtain the number of cells per ml. To differentiate between vital and dead cells, the cells were stained with trypan blue prior to counting. Dead cells appeared blue whereas vital cells did not take up the dye.

### Transformation

### Electroporation of H-CB-P1 Cells:

In a standard electroporation reaction 10 µg of linearised plasmid DNA were used. The culture medium was removed and the H-CB-P1 monolayer washed with citrate buffer and trypsinised (see passage "Trypsinisation" above). The cell pellet was re-suspended in Opti-MEM (pre-warmed to 37°C) to obtain 3 x 10⁶ cells per ml. A volume of 700 µl of the cell suspension was transferred into the electroporation cuvette (peqLab; EQUBIO 4 mm) and the linearised DNA (10 µg) added. The cells were electroporated at 250 V, 1500 µF and immediately afterwards transferred into T75 bottles containing pre-warmed PBG 1.0 medium supplemented with 5% FCS, and incubated at 37°C and 5% CO₂.

### Selection

### Selection of H-CB-P1 Cells:

The electroporated H-CB-P1 cells were cultured for two days at 37°C and 5% CO₂. On day 2, the culture medium was removed and non-adhesive cells harvested by centrifugation of the culture medium. 1 ml of the culture medium (supernatant) was frozen for later examination of transient expression. The trypsinized cell monolayer and the cells harvested from the culture medium by centrifugation were combined and pelleted. Cells were re-suspended in PBG 1.0 medium supplemented with 5% FCS to obtain dilutions of 1x10⁶, 1x10⁵ and 1x10⁴ cells/ml. Each dilution was supplemented with 5 µg/ml or 10 µg/ml blasticidin or with 200 µg/ml or 400 µg/ml hygromycin. The selection medium was changed on days 4, 7 and 10, and the growth of the H-CB-P1 clones controlled via microscopy. Between days 8 and 10 vital clones were visible by eye. On day 13 clones were harvested by trypsinisation, the cell pellet suspended in PBG 1.0 medium so that when cells were seeded into 96 well plates, a well contained either 5 cell or 1 cell. The selection pressure (either 5 or 10 µg/ml blasticidin or 200 or 400 µg/ml hygromycin) was maintained throughout. On day 10 the cells were immuno-stained to differentiate between positive and negative cell clones. The cell culture medium was removed and replaced with standard medium supplemented with a fluorecently labeled antibody (2 µg/ml) recognizing the recombinant protein produced by the cells. The antibody suspension was left for 4 h on the cell monolayer before it was replaced with OptiMem 1 supplemented with 5% FCS. The cell monolayers were examined under a fluorescent microscope. When Texas red conjugated antibodies were used, the microscopy was done with a UV filter (WG) that is transmissible for 470-480 nm spectra. The excited Texas red labeled antibodies emit light in the spectra of 590 nm. A UV filter (WU) transmissible for spectra of 330-355 nm was used for visualization of antibodies conjugated to AMCA. Emission of excited AMCA occurred in the blue spectra (420 nm). Only clones that were large and strongly fluorescent were considered. Was there only a single clone in one well, the cells were further expanded. Was there more than only one clone in a well, the individual clones (cells) were picked with a microcapillar and transferred into a new 96 well plate for further expansion (see the following passage "Mircopillary Picking").

### Microcapillary Picking:

The microcapillary picking device used a capillary attached to a movable arm that was controlled via a joystick. The microcapillar and the arm were inside the hood wheras the joystick was controlled from outside. When an interesting clone was identified via the immunostaining technique (described in section "Selection of H-CB-P1" above), the microcapillary was placed over the clone, negative pressure induced within the capillary through a vacuum pump and the cell pile of interest sucked into the microcapillary. The arm was moved over the fresh well of a 96 well plate and the cells therein ejected.

### Cryoconservation:

For long term storage cells trypsinized cells were re-suspended at 1x10⁶ to 1x10⁷ cells/ml. The cells were pelleted by centrifugation (700 rpm, 10 min) and the supernatant removed. The cells re-suspended in cold pre-conditioned medium (900 µl),a cryo-vial filled with 180 µl DMSO and 720 µl FCS, and the 900 µl cell suspension transferred into the DMSO/FCS solution. The cryo-vial was stored for 24 h in a special freezing container to freeze the cells gently. For long term storage the cryo-vials were transferred into liquid nitrogen tanks (storage at -196°C).

### Detection of Protein Products

### EC-Western-Blot (Enhanced Chemiluminscence):

For detection of hobFC antibodies 20 µl of cell culture supernatant were mixed with 10 µl 5% SDS and incubated for 2 min at 97°C. Was the cell culture medium expected to contain IgM antibodies, the culture medium was not treated. A membrane (Amersham-Pharmacia; Hybond-P) was first rinsed in methanol (1 min), washed three times in water (1 min), and then soaked in plot transfer buffer before placing it on a piece of 3 MM paper also soaked with plot transfer buffer. 5 µl of the pre-treated (hobFC antibodies) or 5 µl of the untreated (IgM antibodies) culture medium were spotted onto the membrane and incubated for 1 min. The membrane was placed in blocking buffer, incubated for half an hour under shaking and three-times washed for 5 min in T-PBS. The blocked membrane was placed in a detecting antibody solution (for IgG 1:2000 and for IgM 1:5000) and incubated for 2 h. Three more washes, of 2, 5 and 10 min in T-PBS buffer followed. The membrane was placed in developer (Amersham-Pharmacia, ECL) and incubated for 1 min. Finally it was drained and placed onto 3 MM paper and wrapped in cellophane to prevent drying. The light emission was observed in a dark room.

### Example 1: Preparation of a targeting vector specific for the IgM region of H-CB-P1 cells

The recombinant gene was to be inserted into the IgM sequence region because it is well-known that antibodies are highly expressed and secreted proteins. The final targeting vector hence required sequences that had 100% homology to the targeted genomic IgM sequences. For the basic targeting vector pVHCµ the VH region of 2 kb and the Cµ intron region of 7.4 kb in length were chosen (see Fig. 3). Both regions were isolated as PCR fragments using polymerases with proofreading activity (proofstart polymerase (Qiagen)), subcloned into a pCR 4BluntTOPO vector (Invitrogen) and finally combined in one vector named pVHCµ (see Fig. 3).

### Preparation of plasmids pVHCµCESHhobFc and pVHCµHhobFc:

The basic targeting vector pVHCµ does not have an endogenous cassette yet. The endogenous cassette containing the CE promoter, the place holder gene hobFc, three FRT recombination sites as well as a hygromycin resistance gene and an ATG deleted neomycin gene was isolated from the vector pCESHhobFc as a BstDI-SwaI fragment. The fragment was then endfilled with Klenow polymerase and ligated into the basic targeting vector pVHCµ that had been digested with PmeI and dephosphorylated. The resulting targeting vector was called pVHCµCESHhobFc.

A second vector pVHCµHhobFc that lacked the CES promoter construct but contained all the other parts of the endogenous cassette was also constructed. A BstBI-Bst1107I fragment was isolated from pCESHhobC and endfilled. The Bstl107I restriction site in pCESHhobFc is immediately upstream of the frt wt site followed by the hobFc gene and thus the isolated Bstl107I-Bstb1 fragment lacks the promoter. The fragment was ligated into a pVHCµ vector previously digested with PmeI and the resulting vector was pVHCµHhobFc. In both vectors pVHCµCSHHobFc and pVHCµHhobFc the active resistance marker gene of the endogenous cassette was the hygromycin resistance gene. An alternative set of vectors that contained a blasticidin gene instead of the hygromycin gene was also created.

### Construction of pVHCµCSHhobFcblas and pVHCµhobFcblas:

To create targeting vectors with the blasticidin gene as marker gene in the endogenous cassette, the vector pcDNATRD was used as donor for the blasticidin gene. The first step involved the exchange of the hygromycin gene with the blasticidin gene. To this end the blasticidin gene was isolated from pcDNATRD as an EcoRI-SaII fragment. The endogenous cassette vector pcESHhobFc was also opened with EcoRI-SalI and thereby the hygromycin gene was removed as well as part of the ATG-deleted neomycin gene. The fragment containing the blastidin gene was ligated into the opened pCESHhobFc and the resulting vector named pCEShobFcblasdeleted.

The second step was the re-insertion of the coincidentally removed ATG-deleted neomycin gene. For that a fragment encompassing the ATG-deleted neomycin gene was isolated from pCESHhobFc and inserted into the opened vector pCEShobFcblasdeleted. The resulting vector was named pCEShobFcblas. This vector now served as donor vector for the blasticidin gene for plasmids pVHCµCSHhobFc and pVHCµHhobFc. A BamHI-SalI fragment was isolated from pCESHhobFcblas and inserted into a BamHI-SalI opened vector pVHCµCESHhobFc, giving rise to pVHCµCEShobFcblas. To create the control vector pVHCµhobFcblas lacking the CES promoter, the vector pVHCµHhobFc was also digested with BamHI-SalI and again the BamHI-SalI fragment isolated from pCEShobFcblas ligated into it.

### Example 2: Selection of hobFc clones

### Electroporation:

H-CB-P1 cells were electroporated with plasmids pVHCµCshobFcblas, pVHCµhobFcblas, pVHCµHhobFc and pCShobFcblas. In order to determine the transfection efficiency, cells were transfected with plasmid pGFPN1VA and as mock control, cells were electroporated with a water sample. The transfection efficiency was found to be at approximately 20%. On day 2 post-electroporation depending on the transfected plasmid either hygromycin or blasticidin was added to the culture medium. When mock-transfected cells were all dead, cells from the other transfection reactions were harvested and re-seeded at a density of either 1 cell or 5 cells per well into a 96 well plate. Cells were continued to be cultured with medium supplemented with the appropriate antibiotic.

To optimise the selection conditions cells were seeded at 10⁴, 10⁵ or 10⁶ cells/20 ml into T75 flasks two days post-electroporation. The medium was supplemented with either 5 or at 10 µg/m blasticidin,or 200 or 400 µg/ml hygromycin. On day 14 the number of clones per cm² was determined. The highest number of clones was obtained in flasks seeded with at 1 x 10⁶ cells that had been transfected with the plasmids lacking the CES promoter (pVHCµhobFcblas). Three times fewer clones were obtained when cells had been transfected with plasmids carrying the CES promoter. Furthermore these clones did grow less well as those without the CES promoter.

### The effect antibiotics have on the selection of protein producing clones:

Following the expansion of the clones in T75 flasks, the clones were trypsinized and re-seeded into 96 well plates at 5 cells/well. The culture medium contained either 5 or 10 µg/ml blasticidin or 200 or 400 µg/ml hygromycin. On day 10 post-seeding, cells were stained with Anti IgG antibodies conjugated with Texas red and AMCA labeled antibodies against IgM. The results obtained with cells cultured with blasticidin showed that with the higher antibiotic concentration far fewer positive clones were obtained. When 10 µg/ml blasticidin were used, 100% more hobFC negative clones were observed compared to 5 µg/ml. However, only the first three rows of the 96 well plate containing cells cultured with 10 µg/ml blasticidin were counted and the result for the entire plate was extrapolated wheras all rows of the 96 well plate containing cells cultured with 5 µg/ml blasticidin where counted.

### Advantage of selection with fluorescently labeled antibodies:

Using the immunofluorescent staining technique large numbers of clones can be screened rapidly. The results obtained with the direct immune-technique were a first indication for correct integration of the targeting sequence into the genomic DNA. Non-expressing clones were easily detected with this technique. The immunofluorescent staining method is technically easy, lower concentrations of antibodies are required than with the methyl-cellulose staining technique, and the proliferation of cells is not impaired. Without any noticeable damages, cells could be immuno-stained before subsequent trypsinization or microcapillary picking to transfer cells into new culture vessels.

### Example 3: Detection of homologous insertion into the IgM locus

For the design of the targeting vector the rearranged immunoglobulin heavy chain locus was assembled based on the cDNA sequence of the antibody and human genome sequence information. To ensure that the production of IgM was disrupted by the targeting approach, the complete leader sequence including the ATG, the V, D and J genes were omitted from the targeting vector and deleted from the genome via a single homologous recombination event. Hence the replacement type targeting vector contained isogenic sequences from the IgM locus to allow the directed cross over as well as the hobFc gene, blasticidin and ATG deleted neomycin resistance genes. Since only one rearranged active IgM locus on chromosome 14 is present in the starting cell line H-CB-P1, the homologous recombination event completely abolishes IgM expression which is detected by fluorescent antibody staining and supernatant immunoblotting using an anti-IgM antibody.

### Western Blot (Dot Blot) for IgM and IgG:

The dot blot technique was used to verify the results obtained with the direct immuno-staining technique. The supernatant (medium) of these clones was examined for presence of IgM and IgG. If supernatant was found to be IgM negative as well as IgG positive, it was concluded that a homologous recombination event had taken place.

### PCR for detection of integrated targeting sequences:

To verify that the targeting cassette had become integrated at the IgM locus PCR reactions were set up with the forward primer V5 (SEQ ID NO:7) and reverse primers V6 (SEQ ID NO:8) or V7 (SEQ ID NO:9), and genomic DNA isolated from cell clones as template. Primer V5 binds to the genomic V gene promoter sequences outside of the fragment Vhprompresent in the targeting vector, reverse primer V6 binds within the CES promoter sequences (and hence was only used for cells transfected with plasmids carrying the CES promoter) and primer V7 bind within the hobFc gene. Using the described primer combinations the occurrence of PCR products is strictly dependent on co-localisation of both primer binding sequences and hence homologous recombination. To increase the sensitivity of this PCR assay, first-round PCR products were used as templates in nested PCR reactions with primers VHpromF (SEQ ID NO:1) and VHpromR (SEQ ID NO:2). Finally nested PCR products were subjected to an enzyme restriction digest with HincII and DraI to confirm that obtained sequences were correct. Passing on all these assays, PBG03 clones H6 (pVHCµhobFcblas), D4 (pVHCµhobFcblas), E11 (pVHCµhobFcblas) D3 (pVHCµCEShobFcblas) and G8 (pVHCµhobfcblas) had integrated the targeting sequence correctly. Clone D3 (pVHCµCEShobFcblas) was renamed PBG04 and deposited at the German Collection of Microorganism and Cell Cultures (DSMZ).

### Example 4: Recombination to generate a target cell clone

Clone pVHCµCEShobFcblas D3 (PBG04) was transfected with vector 2 comprising a second functional sequence (frtwt, GFP ORF and polyadenylation signal, minimal promoter followed by ATG and frt5) and plasmid pflp comprising a functional expression unit for flp recombinase using the transfection reagent effectene (Qiagen). Vector 2 does not contain a promoter driving the GFP expression unit. The functionalised cell (PBG04) is sensitive to Geneticin selection from 200 µg/ml. Vector 2 misses a neomycin resistance gene sequence which could confer resistance to Geneticin. As expected no green fluorescence was detectable 1-4 days after transfection. After two weeks of selection with Geneticin individual stable clones strongly expressing GFP were detectable. GFP expression depends on integration in direct proximity to a functional promoter. Geneticin resistance is dependent on reconstitution of the neo resistance gene present in the cell line by the ATG from vector 2. We conclude that in all cases vector 2 has replaced sequences between the frtw and frt F5 sites and functionally linked the CES promoter and GFP as well as the ATG deleted neomycin gene with the ATG.

## Claims

1. A process for preparing a cell capable of stable high yield expression of a target gene product, which method comprises:
(a) selecting a starting cell capable of stable high yield expression of a starting gene product being non-essential to the starting cell;
(b) screening for the locus of the starting gene product within the genome of the starting cell;
(c1) replacing the gene coding for the starting gene product with a first functional DNA sequence containing one or more recombinase recognition sites (RRS) to obtain a functionalized precursor cell; and
(d) integrating a second functional DNA sequence containing a DNA sequence coding for the target gene product into the functionalized precursor cell obtained in step (c1) by use of a recombinase recognizing the RRSs incorporated with the first functional sequence, or
(c2) directly replacing the gene coding for the starting gene product with a functional DNA sequence containing a DNA sequence coding for the target gene product.

2. The method of claim 1, wherein
(i) the starting cell secretes the starting gene product, preferably in an amount of at least 0.3 fmol/cell/d of a polypeptide chain and more preferably in an amount of more than 1 fmol/cell/d; and/or
(ii) the starting cell is a eukaryotic cell, preferably a primary, immortalized or fusionated cell or a genetic modification thereof; and/or
(iii) the starting gene product is selected from secreted proteins such as antibodies, cytokines, hormones, enzymes, transport proteins, etc.

3. The method of claim 2 wherein the starting cell is selected from primary cells, immortalized cells or tumor cells or genetic modifications thereof, cell hybrids and cell lines used generally in protein manufacturing, preferably is a mammalian hybridoma cell and most preferably is human-mouse hetero-hybridoma H-CB-P1 (DSM ACC 2104).

4. The method according to any one of claims 1 to 3 wherein the locus of the starting gene product is known or is determined by a screening procedure comprising microarray expression analysis, 2D protein gel electrophoresis, quantitative PCR, RNAse protection, northern blot, ELISA, western blot and combinations thereof.

5. The method according to any one of claims 1 to 4, wherein the replacement of the starting gene is effected
(i) by an one step replacement strategy, wherein the starting cell is contacted with a vector construct containing the first functional sequence, said first functional sequence replacing the gene coding for the starting gene product; or
(ii) in a two- or multi-step strategy, wherein the gene coding for the starting gene product is deleted or inactivated and subsequently contacted with a vector construct containing the first functional sequence, said first functional sequence being incorporated at the site of the deleted/inactivated starting gene product.

6. The method of claim 1 or 5, wherein the first functional sequence
(i) comprises one or more RRS(s) selected from loxP, frt, att L and attR sites of lambdoid phages, and recognition sites for resolvases or phage C31 integrase; and/or
(ii) further comprises a functional sequences selected from marker sequences, secretion proteins, promoters, enhancers, splice signals, polyadenylation signals and IRES elements; and/or
(iii) is flanked in the vector by sequences that are homologous to the target gene or adjacent sequences.

7. The method of any one of claims 1 to 6, wherein
(i) the integration of the second functional DNA sequence is effected by delivering a recombinases recognising the RRS(s) present in the first functional sequence together with, shortly before or after delivery of the second functional sequence;
(ii) the integrase is selected from Cre, Flp, φC31 integrase, resolvase, etc.;
(iii) the target gene product is selected from enzymes, hormones, cytokines, receptors, antibodies, antibody domains, fusion proteins comprising the gene product mentioned before, etc.
(iii) the second functional DNA sequence further comprises functional sequences selected from promoter sequences, marker sequences, splice donor and acceptor sequences, recombinase recognition sequences differing from RRS of the first functional sequence, etc.

8. The method of any one of claims 1 to 5, wherein the gene coding for the starting gene product is directly replaced with a functional DNA sequence containing a DNA sequence coding for the target gene product.

9. The method of any one of claims 1 to 8, wherein the starting cell is an immortalized cell derived from B lymphocytes preferably is a myeloma or hybridoma cell, and more preferably a human myeloma or hybridoma and the integration of the functional DNA sequence(s) is effected at a rearranged Ig locus of said cell.

10. The method of any one of claims 9, wherein the starting cell is a human-mouse hetero-hybridoma, preferably is hetero-hybridoma H-CB-P1 (DSM ACC 2104), and the integration of the functional DNA sequence(s) is effected at the rearranged immunoglobulin H locus or **λ** locus of said cell.

11. A cell capable of high yield expression of a target gene product obtainable by the method of claims 1 to 10.

12. A method for preparing a functionalized cell comprising the steps (a) to (c1) as defined in claims 1 to 5.

13. A functionalized cell as defined in claim 12.

14. A method for high yield expression of a target gene product which comprises cultivating a cell as defined in claim 11.


SEQUENCE LISTING

<110> ProBioGen AG

<120> Creation of High Yield Heterologous Expression Cell
      Lines

<130> 021802ep JH/BM

<160> 12

<170> PatentIn Ver. 2.1

<210> 1
<211> 33
<212> DNA
<213> Artificial Sequence

<220>
<223> Description of Artificial Sequence: Primer VHpromF

<400> 1
atactagtcg gccgcaggca catccacagt cac                                33

<210> 2
<211> 32
<212> DNA
<213> Artificial Sequence

<220>
<223> Description of Artificial Sequence: Primer VHpromR

<400> 2
tcccgggtat cgatggagct ctcaggggat tc                                 32

<210> 3
<211> 27
<212> DNA
<213> Artificial Sequence

<220>
<223> Description of Artificial Sequence: Primer CμintV

<400> 3
catcgatccg ctactactac tacatgg                                       27

<210> 4
<211> 18
<212> DNA
<213> Artificial Sequence

<220>
<223> Description of Artificial Sequence: Primer CμintR

<400> 4
cggccacgct gctcgtat                                                 18

<210> 5
<211> 18
<212> DNA
<213> Artificial Sequence

<220>

<223> Description of Artificial Sequence: Primer
      CμMitteR

<400> 5
agctcacctg gtgcaact                                                    18


<210> 6
<211> 19
<212> DNA
<213> Artificial Sequence

<220>
<223> Description of Artificial Sequence: primer
      CμMitteF

<400> 6
gacctaagct gacctagac                                                   19


<210> 7
<211> 18
<212> DNA
<213> Artificial Sequence

<220>
<223> Description of Artificial Sequence: Primer V5

<400> 7
tccctccaaa agctgtag                                                    18


<210> 8
<211> 18
<212> DNA
<213> Artificial Sequence

<220>
<223> Description of Artificial Sequence: primer V6

<400> 8
atggcggtaa tgttggac                                                    18


<210> 9
<211> 18
<212> DNA
<213> Artificial Sequence

<220>
<223> Description of Artificial Sequence: primer V7

<400> 9
cacaagaatc cgcacagg                                                    18


<210> 10
<211> 18
<212> DNA
<213> Artificial Sequence

<220>
<223> Description of Artificial Sequence: Primer
      EBVtestR

<400> 10
cctgatattg caggtagg                                                    18

<210> 11
<211> 18
<212> DNA
<213> Artificial Sequence

<220>
<223> Description of Artificial Sequence: Primer
      EBVtestF

<400> 11
taccgacgaa ggaacttg                                                18


<210> 12
<211> 377
<212> DNA
<213> Artificial Sequence

<400> 12
cagctggtgc agtctggggc tgaggtgaag aagcctgggg cctcagtgaa ggtctcctgc 60
aaggcttctg gatacacctt caccggctcc tatatgcact gggtgcgaca ggcccctgga 120
caaggccttg agtggatggg acggatcaat cctaacagtg gtggcacaaa ctatgcacag 180
aaatttcagg gcagggtcac catgaccagg gacacgtcca tcagcacagc ctacatggag 240
ctgagcaggc tgagatctga cgacacggcc gtgtattact gtgcgagaga caagctttcc 300
cggtcagaag taccagctgg ccgctactac tactacatgg acgtctgggg caaagggacc 360
acggtcaccg tctcctc                                                 377